(19) Europäisches Patentamt
European Patent Office
Office européen des brevets

(11) Veröffentlichungsnummer: **0 195 251**
**A2**

(12) **EUROPÄISCHE PATENTANMELDUNG**

(21) Anmeldenummer: **86101964.4**

(22) Anmeldetag: **17.02.86**

(51) Int. Cl.4: **A 61 K 7/08**
**A 61 K 7/06**

(30) Priorität: **25.02.85 DE 3506543**

(43) Veröffentlichungstag der Anmeldung:
**24.09.86 Patentblatt 86/39**

(84) Benannte Vertragsstaaten:
**AT BE CH DE FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Höffkes, Horst, Dr.**
**Carlo-Schmid-Strasse 113**
**D-4000 Düsseldorf-Hellerhof(DE)**

(54) **Haarspülmittel mit Perlglanzeffekt.**

(57) Haarspülmittel mit einem Gehalt an kationischen Tensiden enthalten zur Bildung eines perlglänzenden Aussehens 0,5 – 5 Gew.–% eines Esters der allgemeinen Formel $R-(OC_nH_{2n})_xOR^2$, in der $R^1$ ein linearer Fettacylrest mit 16 – 22 C–Atomen, $R^2$ Wasserstoff oder eine Gruppe $R^1$ und n = 2 oder 3 und x eine Zahl von 1 – 4 ist.
0,1 – 3 Gew.–% eines Monoethanolamids, einer Fettsäure mit 12 – 18 C-Atomen und
0,05 – 2 Gew.–% eines nichtionogenen Ethylenoxidaddukts mit einem HLB–Wert von 4 – 12.
Bevorzugt ist zusätzlich 1 – 5 Gew.–% eines Fettalkohols mit 14 – 22 C-Atomen oder eines Fettsäuremono- und/oder diglycerids einer Fettsäure mit 16 – 22 C-Atomen enthalten.

EP 0 195 251 A2

Henkelstraße 67
4000 Düsseldorf, den 09.09.1985

JG/Po

Patentanmeldung

D 7254 EP

"Haarspülmittel mit Perlglanzeffekt"

Gegenstand der Erfindung sind Mittel zum Spülen von Haaren mit einem Gehalt an kationischen Tensiden, und mit einer speziellen Kombination von Hilfsmitteln, die den Haarspülmitteln ein perlglänzendes Aussehen verleihen.

Haarspülmittel werden angewendet, um dem Haupthaar, insbesondere nach dem Waschen, Bleichen oder Färben der Haare eine verbesserte Kämmbarkeit, erhöhten Glanz, eine verringerte Aufladbarkeit und der Frisur mehr Halt und Fülle zu verleihen. Solche Haarspülmittel enthalten als Wirkstoffe mit substantiver, antistatischer Wirkung kationische Tenside, bevorzugt vom Typ der quartären Ammoniumverbindungen. Geeignete kationische Tenside werden z.B. im "Handbuch der Kosmetika und Riechstoffe" von Hugo Janistyn, III. Band, 2. Auflage (1973) auf Seite 419 - 420 genannt. Zur Behebung der statischen Aufladbarkeit genügt es, wäßrige Lösungen solcher kationischer Tenside auf das Haar aufzubringen. Es ist jedoch erwünscht, daß solche Haarspülmittel einen zusätzlichen avivierenden Effekt - zur Verbesserung von Glanz und Fülle des Haares - aufweisen und daß sie ein cremiges, gehaltvolles Aussehen haben. Dies läßt sich dadurch erreichen, daß Fettstoffe, z.B. Fettalkohole, Fettsäureglyceri-

...

de, Fettsäureester oder Paraffine in emulgierter Form in die Haarspülmittel eingesetzt werden.

Die emulsionsförmigen Haarnachspülmittel, z.B. solche mit einem Gehalt an Cetyl- und/oder Stearylalkohol wirken aber wenig ansprechend, sie sehen durchscheinend aus und sind wenig cremig. Es ist auch bekannt, solchen Präparaten Perlglanz-Präparate zuzugeben, um das Aussehen zu verbessern. Aus "Parfümerie und Kosmetik", 60 Jahrgang, Nr. 10 (1979), Seite 351 - 353 ist bekannt, daß Ethylenglykolester und Monoethanolamide höherer Fettsäuren nicht den gewünschten Effekt bewirken. Dort wird vorgeschlagen, 90 %iges Glycerinmonostearat zusammen mit einem Coemulgator, der einen HLB-Wert von 15 - 17 aufweist, zur Perlglanzbildung zu verwenden.

Es hat sich aber gezeigt, daß solche Haarspülmittel nur eine unbefriedigende Verbesserung der Naßkämmbarkeit und der Haaravivage, insbesondere bei strapaziertem Haar aufweisen. Es bestand daher die Aufgabe, Formulierungsmöglichkeiten für Haarspülungen aufzufinden, die sowohl einen stabilen, homogen verteilten Perlglanz als auch eine hohe Haaravivagewirkung aufweisen.

Es wurde gefunden, daß die gestellte Aufgabe durch Zusatz einer Kombination aus

0,5 - 5 Gew.-% eines Esters der allgemeinen Formel $R^1(OC_nH_{2n})_xOR^2$, in der $R^1$ ein linearer Fettsäureacylrest mit 16 - 22 C-Atomen, $R^2$ Wasserstoff oder eine Acylgruppe $R^1$, n = 2 oder 3 und x eine Zahl von 1 - 4 ist.

0,1 - 3 Gew.-% eines Monoethanolamids einer Fettsäure mit 12 - 18 C-Atomen und

. . .

0,05- 2 Gew.-% eines nichtionogenen Ethylenoxidadduktes mit einem HLB-Wert von 4 - 12

bezogen auf das gesamte Haarspülmittel erreicht wird.

Als Ester der allgemeinen Formel $R^1(OC_nH_{2n})_xOR^2$ können z.B. die Mono- und Diester des Ethylenglykols und Propylenglykols mit höheren Fettsäuren, z.B. mit Palmitinsäure, Stearinsäure oder Behensäure oder die Diester des Diethylenglykols oder des Triethylenglykols mit solchen Fettsäuren eingesetzt werden. Geeignet sind auch Mischungen von Mono- und Diestern der genannten Glykole mit Fettsäuregemischen, z.B. mit gehärteter Talgfettsäure oder mit der gesättigten $C_{16}$-$C_{18}$-Fettsäurefraktion der Talgfettsäure. Bevorzugt geeignet ist der Ethylenglykolmono- und/oder Diester der Palmitin- und/oder Stearinsäure.

Als Monoethanolamide von Fettsäuren mit 12 - 18 C-Atomen können z.B. Laurinsäuremonoethanolamid, Myristinsäuremonoethanolamid, Palmitin-/Stearinsäure-Monoethanolamid und bevorzugt das Monoethanolamid der $C_{12}$-$C_{18}$-Fraktion der Kokosölfettsäure verwendet werden.

Als nichtionogene Ethylenoxidaddukte eignen sich die Anlagerungsprodukte von Ethylenoxid an Fettalkohole, Fettsäuren und Fettsäureamide oder Alkanolamide, wobei der Fettalkyl- oder Fettacylrest bevorzugt 12 - 22 C-Atome trägt. Weiterhin eignen sich Ethylenoxidaddukte an Alkylphenole, bevorzugt an solche mit einer Alkylgruppe mit 8 - 16 C-Atomen. Schließlich eignen sich auch Ethylenoxidaddukte an Fettsäure-Polyol-Partialester, z.B. an Glycerinmonoester, an Pentaerythritmonoester, Sorbitanmono- und -diester von Fettsäuren mit bevorzugt 12 - 22 C-Atomen. Das Gewichtsverhältnis von hydrophilen zu lipophilen Gruppen in diesen Ethylenoxidaddukten sollte so sein, daß das Gewicht der hydrophilen Gruppen, also das Gewicht der (vom Ethylenoxid gebildeten) Polyethylenglykolethergruppen und der (bei

...

Fettsäure-Polyol-Partialester-Addukten) Polyolgruppen etwa 20 - 60 Gew.-% des Gesamtmoleküls der Ethylenoxidaddukte ausmacht. Bei diesen für die Herstellung erfindungsgemäßer Haarspülmittel geeigneten Ethylenoxidaddukten liegt der HLB-Wert gemäß HLB = $\frac{E + P}{5}$ (wobei E = Gehalt Ethylenoxid in Gew.-% und P = Gehalt an mehrwertigem Alkohol in Gew.-% im Addukt), also im Bereich von 4 - 12.

Besonders gut ausgeprägten Perlglanz von guter Stabilität zeigen erfindungsgemäße Haarspülmittel, die einen Gehalt von

1,0 - 2,5 Gew.-% eines Ethylenglykolmono- und/oder Diesters der Palmitin- und/oder Stearinsäure,

0,4 - 1,5 Gew.-% eines Kokosfettsäure-($C_{12}$-$C_{18}$)-monoethanolamids

0,05 - 2 Gew.-% eines Adduktes von 3 - 6 Mol Ethylenoxid an einen Fettalkohol mit 12 - 18 Fettalkoholen

aufweisen. Die Zugabe der zur Perlglanzbildung geeigneten und für die Erfindung kennzeichnenden Kombination zu dem Haarspülmittel erfolgt bevorzugt in Form eines separat hergestellten Konzentrates. Ein solches Konzentrat kann z.B. 5 - 30 Gew.-% des Esters der allgemeinen Formel $R^1(OC_nH_{2n})_xOR^2$, 2 - 10 Gew.-% eines Fettsäuremonoethanolamids und 3 - 20 Gew.-% des nichtionogenen Ethylenoxidadduktes mit einem HLB-Wert von 4 - 12, bezogen auf das gesamte Konzentrat, enthalten.

Die Herstellung dieses Konzentrats erfolgt zweckmäßigerweise dadurch, daß die genannten Komponenten gemeinsam aufgeschmolzen und auf eine Temperatur von 70 - 90°C erwärmt werden und dann Wasser mit einer Temperatur von 70 - 90°C in der berechneten Menge portionsweise unter Rühren zugemischt wird. Dabei entsteht eine Emulsion, die bei langsamem Abkühlen unter Rühren in eine perlglänzende Dispersion übergeht. Diese Dis-

. . .

persion kann dann bei der Herstellung des Haarspülmittels während oder nach dem Abkühlen der Lösung oder Emulsion der übrigen Komponenten oder nach dem Abkühlen zugesetzt werden.

Wie weiter oben ausgeführt, enthalten die erfindungsgemäßen Haarspülmittel kationische Tenside als Wirkstoffe mit substantiver, antistatischer Wirkung. Besonders zweckmäßig ist ein Gehalt von 0,5 - 5 Gew.-% eines kationischen Tensids der allgemeinen Formel II

(II) 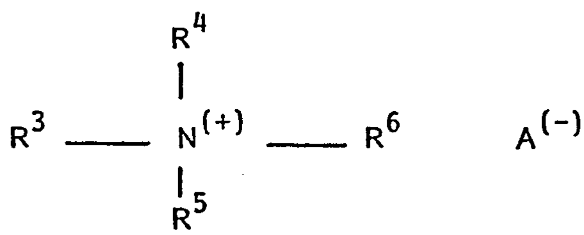

in der $R^3$ eine Alkyl- oder Hydroxyalkylgruppe mit 8 - 22 C-Atomen, eine Gruppe $R^7-CONH(CH_2)_y-$, in der $R^7$ eine Alkylgruppe mit 7 - 21 C-Atomen und y eine Zahl von 2 - 4 ist, $R^4$ und $R^5$ eine Alkylgruppe mit 1 - 4 C-Atomen oder eine Gruppe der Formel $-(C_mH_{2m}O)_zH$, in der m eine Zahl von 2 - 4 und z eine Zahl von 1 - 10 ist und $R^6$ eine Benzylgruppe ist oder eine der für $R^3$, $R^4$ und $R^5$ angegebenen Bedeutungen hat, und $A^{(-)}$ ein Chlorid-, Bromid-, Hydrogensulfat, Hydrogenphosphat, Methoxysulfat oder Ethoxysulfatanion ist.

Geeignete kationische Tenside sind z.B. Cetyltrimethylammoniumchlorid, Lauryldimethylbenzylammoniumchlorid, Stearyltrimethylammoniumchlorid, Stearyldimethylbenzylammoniumchlorid, Distearyldimethylammoniumchlorid, Talgalkyl-tris-(oligooxyethyl)-ammonium-phosphat, 2-Hydroxyhexadecyl-2-hydroxyethyl-dimethylammoniumchlorid und 2-Hydroxyhexadecyl-bis-(2-hydroxyethyl)-methylammoniumchlorid.

Zur Verbesserung der haaravivierenden Wirkung ist es weiterhin zweckmäßig, daß die erfindungsgemäßen Haarspülungen zusätz-

. . .

lich einen Fettstoff, z.B. eine kosmetische Öl-, Fett- oder Wachskomponente, ein Paraffinöl- oder Paraffinwachs, Wollwachs oder ein Wollwachsderivat enthalten. Als Fettstoff sind besonders Fettalkohole mit 14 - 22 C-Atomen, z.B. Cetyl- und/oder Stearylalkohol sowie Fettsäuremonoglyceride und/oder Fettsäurediglyceride von Fettsäuren mit 16 - 22 C-Atomen, z.B. von Palmitin- und/oder Stearinsäure geeignet. Solche Fettstoffe sind bevorzugt in Mengen von 1 - 5 Gew.-%, bezogen auf die gesamte Zubereitung enthalten.

Außer den genannten Komponenten können die erfindungsgemäßen Haarspülmittel noch weitere, an sich in Haarspülmitteln übliche Hilfs- und Zusatzmittel in untergeordneten Mengen bis etwa 5 Gew.-% enthalten. Solche Hilfs- und Zusatzmittel sind z.B. kationische Polymere, etwa vom Typ der quartären Cellulosederivate, die unter der Handelsbezeichnung Polymer JR 400 erhältlich sind oder andere kationische Polymere, wie sie z.B. aus US-PS 4.240.450 bekannt sind.

Weitere übliche Zusatzmittel sind niedere Polyole, z.B. 1.2-Propylenglykol oder Glycerin, wasserlösliche nichtionogene Verdickungsmittel wie z.B. Hydroxyethylcellulose, Methylhydroxypropylcellulose, Hydroxyethylstärke, Hydroxypropylguar, Konservierungsstoffe, Duftstoffe, Farbstoffe, Lichtschutzmittel sowie haarkosmetische Wirkstoffe wie z.B. Vitamine, Pflanzenextrakte, Balsame, Antischuppenwirkstoffe (z.B. Zn- oder Mg-Pyridinthion) oder Sebostatika.

Die Herstellung der erfindungsgemäßen Haarspülmittel erfolgt durch Aufschmelzen und Erwärmen der Fettkomponenten auf 70 - 90°C und portionsweise Einarbeitung unter Rühren der wäßrigen Lösung des kationischen Tensids. Während des Abkühlens der Emulsion, etwa bei 40°C können die sonstigen wasserlöslichen Hilfs- und Zusatzmittel zugesetzt werden. Die nichtionogenen Verdickungsmittel werden bevorzugtin Form einer wäßrigen Lösung zugesetzt.

. . .

Die für die Erfindung kennzeichnenden Komponenten können entweder gemeinsam mit den ggf. sonstigen Fettkomponenten aufgeschmolzen und eingearbeitet werden. Bevorzugt wird jedoch, wie weiter oben beschrieben, ein Perlglanzmittel-Konzentrat in Form einer fließfähigen Dispersion in Wasser hergestellt und dieses Konzentrat der Lösung oder Emulsion der übrigen Komponenten während des Abkühlvorganges etwa bei 40°C oder nach dem Erkalten zugesetzt.

Die folgenden Beispiele sollen den Gegenstand der Erfindung näher erläutern ohne ihn hierauf zu beschränken.

...

## Beispiele

### 1. Perlglänzendes Haarnachspülmittel

### 1.1 Herstellung eines Perlglanzkonzentrats

Zusammensetzung

| | |
|---|---|
| Gemisch aus 35 Gew.-% Ethylenglykolmonoester und 65 Gew.-% Ethylenglykoldiester eines Palmitin/Stearinsäure (1 : 1)-Schnittes | 18 Gew.-% |
| Monoethanolamid eines Kokosfettsäure-$C_{12}$-$C_{18}$-Schnittes | 6 Gew.-% |
| Addukt von 4 Mol Ethylenoxid an einen $C_{12}/C_{14}$-(7 : 3)-Fettalkoholschnitt | 10 Gew.-% |
| Wasser | 66 Gew.-% |

Das Glykolstearat/palmitat, das Fettsäurealkanolamid und der Fettalkoholpolyglykolether werden zusammen aufgeschmolzen und die Schmelze bei 90°C homogenisiert. In die Schmelze wird das 80°C heiße Wasser unter Rühren portionsweise eingearbeitet. Die fertige Emulsion wird unter langsamem Rühren auf Raumtemperatur abgekühlt.

...

1.2 Perlglänzendes Haarnachspülmittel

| | |
|---|---|
| Cetyltrimethylammonium-chlorid (25 Gew.-% in Wasser) | 2,0 Gew.-% |
| Fettalkoholschnitt $C_{16}/C_{18}$ (1:1) | 3,0 Gew.-% |
| Perlglanzkonzentrat nach 1.1 | 10,0 Gew.-% |
| Parfümöl | 0,2 Gew.-% |
| Wasser | 94,8 Gew.-% |

Der Fettalkohol $C_{16}/C_{18}$ wird auf ca. 80°C erhitzt und die auf ca. 80°C erwärmte Lösung von Cetyltrimethylammoniumchlorid in Wasser in den geschmolzenen Fettalkohol einemulgiert. Nach dem Abkühlen auf ca. 40°C wird das Perlglanzkonzentrat eingemischt, nach weiterem Abkühlen auf ca. 30°C das Parfümöl zugesetzt.

. . .

## Patentansprüche

1. Haarspülmittel mit Perlglanzeffekt mit einem Gehalt an kationischen Tensiden und üblichen Hilfs- und Zusatzmitteln, dadurch gekennzeichnet, daß zur Bildung eines perlglänzenden Aussehens

0,5 - 5 Gew.-% eines Esters der allgemeinen Formel

$$R^1(OC_nH_{2n})_xOR^2$$

in der $R^1$ ein linearerer Fettsäureacylrest mit 16 - 22 C-Atomen, $R^2$ Wasserstoff oder eine Gruppe $R^1$ und n = 2 oder 3 und x eine Zahl von 1 - 4 ist.

0,1 - 3 Gew.-% eines Monoethanolamids einer Fettsäure mit 12 - 18 C-Atomen

0,05 - 2 Gew.-% eines nichtionogenen Ethylenoxidadduktes mit einem HLB-Wert von 4 - 12,

bezogen auf das gesamte Mittel, enthalten sind.

2. Haarspülmittel nach Anspruch 1, dadurch gekennzeichnet, daß der Ester ein Ethylenglykolmono- und/oder Diester der Palmitin- und/oder Stearinsäure ist.

3. Haarspülmittel nach Anspruch 1 oder 2, dadurch gekennzeichnet, daß

1,0 - 2,5 Gew.-% eines Ethylenglykolmono- und/oder Diesters der Palmitin- und/oder Stearinsäure

. . .

0,4 - 1,5 Gew.-%eines Kokosfettsäure($C_{12}$-$C_{18}$)monoethanolamid

0,05 - 2,0 Gew.-%eines Adduktes von 3 - 6 Mol Ethylenoxid an einen Fettalkohol mit 12 - 18 C-Atomen

enthalten ist.

4. Haarspülmittel nach Anspruch 1 -3, dadurch gekennzeichnet, daß

0,5 - 5 Gew.-% eines kationischen Tensids der allgemeinen Formel II

(II) 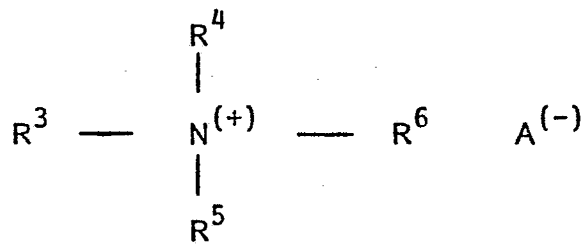

in der $R^3$ eine Alkyl- oder Hydroxyalkylgruppe mit 8 - 22 C-Atomen, eine Gruppe $R^7CONH(CH_2)_y$, in der $R^7$ eine Alkylgruppe mit 7 - 21 C-Atomen und y eine ganze Zahl von 2 - 4 ist, $R^4$ und $R^5$ eine Alkylgruppe mit 1 - 4 C-Atomen oder eine Gruppe der Formel - $(C_mH_{2m}O)_zH$, in der m eine Zahl von 2 - 4 und z eine Zahl von 1 - 10 ist, und $R^6$ eine Benzylgruppe ist oder eine der für $R^3$, $R^4$ und $R^5$ angegebenen Bedeutungen hat, und $A^{(-)}$ ein Chlorid-, Bromid-, Hydrogensulfat-, Hydrogenphosphat, Methoxysulfat- oder Ethoxysulfatanion ist, enthalten sind.

5. Haarspülmittel nach Anspruch 1 - 4, dadurch gekennzeichnet, daß zusätzlich

. . .

1 - 5 Gew.-% eines Fettalkohols mit 14 - 22 C-Atomen oder eines Fettsäuremono- und/oder -diglycerids einer Fettsäure mit 16 - 22 C-Atomen

enthalten ist.

6. Perlglanzkonzentrat zur Herstellung von Haarspülmitteln gemäß Anspruch 1, dadurch gekennzeichnet, daß es

5 - 30 Gew.-% eines Esters der allgemeinen Formel $R^1 (OC_nH_{2n})_xOR^2$, in der $R^1$, $R^2$, n und x die in Anspruch 1 angegebene Bedeutung haben,

2 - 10 Gew.- %eines Fettsäuremonoethanolamids einer Fettsäure mit 12 - 18 C-Atomen und

3 - 30 Gew.-% eines nichtionogenen Ethylenoxidaddukts mit einem HLB-Wert von 4 - 12

bezogen auf das gesamte Konzentrat enthält.

...